# EUROPEAN PATENT SPECIFICATION

(11) **EP 3 475 250 B1**
(45) Date of publication and mention of the grant of the patent: **16.12.2020**
(21) Application number: 17729686.0
(22) Date of filing: 01.06.2017
(51) Int. Cl.: C07C 2/62, C07C 9/16, C07C 9/21

(54) **ISOPARAFFIN-OLEFIN ALKYLATION**
ISOPARAFFIN-OLEFIN-ALKYLIERUNG
ALKYLATION D'UNE OLÉFINE PAR UNE PARAFFINE

(30) Priority: 23.06.2016 US 201662353666 P; 23.06.2016 US 201662353671 P; 23.06.2016 US 201662353687 P; 23.06.2016 US 201662353675 P; 23.06.2016 US 201662353684 P
(43) Date of publication of application: 01.05.2019
(73) Proprietor: ExxonMobil Research and Engineering Company, Annandale, NJ 08801-0900 (US)
(72) Inventor: CHOUDHARY, Vinit, Annandale, NJ 08801 (US); DAKKA, Jihad, M., Whitehouse Station, NJ 08889 (US); METTLER, Matthew, S., Somerville, NJ 08876 (US); JOHNSON, Ivy, D., Lawrenceville, NJ 08648 (US); ALLEN, Joshua, W., Branchburg, NJ 08876 (US); DANDEKAR, Ajit, B., Spring, TX 77389-1425 (US); OMILIAN, Cynthia, F., Whitehouse Station, NJ 08889 (US)
(74) Representative: ExxonMobil Chemical Europe Inc.
(86) International application number: PCT/US2017/035363
(87) International publication number: WO 2017/222770

(56) References cited:
- WO-A1-93/07106
- WO-A1-2015/112293
- US-A- 3 865 894
- US-A- 5 258 569
- US-A- 5 304 698
- US-A- 5 420 093
- US-A- 5 625 113
- US-A- 5 780 703
- US-B1- 6 756 030
- US-B1- 7 982 084

## Description

### FIELD

The present disclosure relates to a process for isoparaffin-olefin alkylation.

### BACKGROUND

Alkylation is a reaction in which an alkyl group is added to an organic molecule. Thus an isoparaffin can be reacted with an olefin to provide an isoparaffin of higher molecular weight. Industrially, the concept depends on the reaction of a C₂ to C₅ olefin with isobutane in the presence of an acidic catalyst producing a so-called alkylate. This alkylate is a valuable blending component in the manufacture of gasoline due not only to its high octane rating but also to its sensitivity to octane-enhancing additives.

Industrial alkylation processes have historically used hydrofluoric or sulfuric acid catalysts under relatively low temperature conditions. The sulfuric acid alkylation reaction is particularly sensitive to temperature, with low temperatures being favored to minimize the side reaction of olefin polymerization. Acid strength in these liquid acid catalyzed alkylation processes is preferably maintained at 88 to 94 weight percent by the continuous addition of fresh acid and the continuous withdrawal of spent acid. The hydrofluoric acid process is less temperature sensitive and the acid is easily recovered and purified.

Both sulfuric acid and hydrofluoric acid alkylation share inherent drawbacks including environmental and safety concerns, acid consumption, and sludge disposal. Research efforts have been directed to developing alkylation catalysts which are equally as effective as sulfuric or hydrofluoric acids but which avoid many of the problems associated with these two acids. For a general discussion of sulfuric acid alkylation, see the series of three articles by L. F. Albright et al., "Alkylation of Isobutane with C4 Olefins", 27 Ind. Eng. Chem. Res., 381-397, (1988). For a survey of hydrofluoric acid catalyzed alkylation, see 1 Handbook of Petroleum Refining Processes 23-28 (R. A. Meyers, ed., 1986). A general overview of the technology can be found in "Chemistry, Catalysts and Processes of Isoparaffin-Olefin Alkylation - Actual Situation and Future Trends, Corma et al., Catal. Rev. - Sci. Eng. 35(4), 483-570 (1993).

With increasing demands for octane and increasing environmental concerns, it is desirable to develop an alkylation process employing safer, more environmentally acceptable catalyst systems. Specifically, it is desirable to provide an industrially viable alternative to the currently used hydrofluoric and sulfuric acid alkylation processes. Consequently, substantial efforts have been made to develop a viable isoparaffin-olefin alkylation process which avoids the environmental and safety problems associated with sulfuric and hydrofluoric acid alkylation while retaining the alkylate quality and reliability characteristics of these well-known processes. Research efforts have therefore for some time been directed towards solid, instead of liquid, alkylation catalyst systems.

For example, U.S. Patent No. 3,644,565 discloses alkylation of a paraffin with an olefin in the presence of a catalyst comprising a Group VIII noble metal present on a crystalline aluminosilicate zeolite having pores of substantially uniform diameter from about 4 to 18 angstrom units and a silica to alumina ratio of 2.5 to 10, such as zeolite Y. The catalyst is pretreated with hydrogen to promote selectivity.

However, the development of a satisfactory solid acid replacement for hydrofluoric and sulfuric acid has proved challenging. For example, U.S. Patent No. 4,384,161 describes a process of alkylating isoparaffins with olefins to provide alkylate using a large-pore zeolite catalyst capable of absorbing 2,2,4-trimethylpentane, for example, ZSM-4, ZSM-20, ZSM-3, ZSM-18, zeolite Beta, faujasite, mordenite, zeolite Y and the rare earth metal-containing forms thereof, and a Lewis acid such as boron trifluoride, antimony pentafluoride or aluminum trichloride. The addition of a Lewis acid is reported to increase the activity and selectivity of the zeolite, thereby effecting alkylation with high olefin space velocity and low isoparaffin/olefin ratio. According to the '161 patent, problems arise in the use of solid catalysts alone in that they appear to age rapidly and cannot perform effectively at high olefin space velocity.

As new solid acid catalysts have become available, they have been routinely screened for their efficacy in isoparaffin-olefin alkylation. For example, U.S. Patent No. 5,304,698 describes a process for the catalytic alkylation of an olefin with an isoparaffin comprising contacting an olefin-containing feed with an isoparaffin-containing feed with a crystalline microporous material selected from the group consisting of MCM-22, MCM-36, and MCM-49 under alkylation conversion conditions of temperature at least equal to the critical temperature of the principal isoparaffin component of the feed and pressure at least equal to the critical pressure of the principal isoparaffin component of the feed. U.S. Patent No. 3,865,894 describes the preparation of olefin-paraffin alkylate by contacting a C₃-C₉ monoolefin with C₄-C₆ isoparaffin with an anhydrous acidic crystalline aluminosilicate zeolite.

Despite these advances, there remains a need for an improved isoparaffin-olefin alkylation process that is catalyzed by a solid acid catalyst but approaches or exceeds the activity and product quality of existing liquid phase processes.

### SUMMARY

Provided herein are processes for olefin/isoparaffin alkylation and alkylated hydrocarbon products. The present invention provides a process for olefin/isoparaffin alkylation, wherein the process comprises, contacting an olefin-containing feed with an isoparaffin-containing feed under alkylation conditions in the presence of a solid acid catalyst comprising a crystalline microporous material of the MWW framework type, wherein the olefin-containing feed consists essentially of pentenes, such as n-pentene, iso-pentene (e.g. 2-methyl-2-butene), or a combination thereof.

In one aspect, the solid acid catalyst comprises an inorganic oxide binder. In another aspect, at least one of alumina, silica, titania, and zirconia binder. The MWW framework type may be selected from the group consisting of MCM-22, PSH-3, SSZ-25, ERB-1, ITQ-1, ITQ-2, MCM-36, MCM-49, MCM-56, EMM-10, EMM-12, EMM-13, UZM-8, UZM-8HS, UZM-37, MIT-1, and mixtures thereof. In certain embodiments, the MWW framework type comprises MCM-49. In other aspects, the isoparaffin-containing feed comprises at least one C₄ to C₈ isoparaffin, e.g. isobutene.

The present invention further provides a hydrocarbon product produced by the process described herein. The product comprises a C₈ fraction comprising trimethylpentane isomers and dimethylhexane isomers and a C₉ fraction comprising trimethylhexane isomers and dimethylheptane isomers, wherein the ratio of trimethylpentane isomers to dimethylhexane isomers is from 3:1 to 22:1 in the C₈ fraction, wherein the ratio of trimethylhexane isomers to dimethylheptane isomers is from 0.5:1 to 2:1 in the C₉ fraction. In certain aspects, the hydrocarbon product comprises a C₅ fraction that makes up between 18-45 wt% of the total C₅₊ liquids in the hydrocarbon product and/or C₉ fraction that makes up between 10-50 wt% of the total C₅₊ liquids in the hydrocarbon product.

### BRIEF DESCRIPTION OF THE DRAWINGS

Figure 1 is a graph of C₅ olefin conversion against days on oil for the MCM-49 catalyst of Example 1 in the alkylation of a premixed isobutane/pentene feed at various liquid hourly space velocity (LHSV) values according to the process of Example 2.
Figure 2 is a graph of product yield as a percentage of total C₅+ hydrocarbons against time on stream obtained according to the process of Example 2.
Figure 3 is a graph of the ratio of trimethyl : dimethyl isomers in the product yield for C₇, C₈, and C₉ hydrocarbons against time on stream according to the process of Example 2.
Figure 4 is a graph of the octane number against time on stream for the total product obtained according to the process of Example 2.
Figure 5 is a graph of C₅ olefin conversion against days on oil for the MCM-49 catalyst of Example 1 in the alkylation of a premixed isobutane/pentene feed according to the process of Example 3 (n-pentene) and Example 4 (2-methyl-2-butene).
Figure 6 is a graph of product yield as a percentage of total C₅+ hydrocarbons against time on stream obtained according to the process of Example 3.
Figure 7 is a graph of product yield as a percentage of total C₅+ hydrocarbons against time on stream obtained according to the process of Example 4.
Figure 8 is a graph of the octane number against time on stream for the total product obtained according to the process of Example 3 (n-pentene) and Example 4 (2-methyl-2-butene).

### DETAILED DESCRIPTION OF THE EMBODIMENTS

Disclosed herein is a process for isoparaffin-olefin alkylation, in which an olefin-containing feed is contacted with an isoparaffin-containing feed under alkylation conditions in the presence of a solid acid catalyst which comprises a crystalline microporous material of the MWW framework types.

As used herein, the term "crystalline microporous material of the MWW framework type" includes one or more of:
- molecular sieves made from a common first degree crystalline building block unit cell, which unit cell has the MWW framework topology. (A unit cell is a spatial arrangement of atoms which if tiled in three-dimensional space describes the crystal structure. Such crystal structures are discussed in the "Atlas of Zeolite Framework Types", Fifth edition, 2001;
- molecular sieves made from a common second degree building block, being a 2-dimensional tiling of such MWW framework topology unit cells, forming a monolayer of one unit cell thickness, preferably one c-unit cell thickness;
- molecular sieves made from common second degree building blocks, being layers of one or more than one unit cell thickness, wherein the layer of more than one unit cell thickness is made from stacking, packing, or binding at least two monolayers of MWW framework topology unit cells. The stacking of such second degree building blocks can be in a regular fashion, an irregular fashion, a random fashion, or any combination thereof; and
- molecular sieves made by any regular or random 2-dimensional or 3-dimensional combination of unit cells having the MWW framework topology.

Crystalline microporous materials of the MWW framework type include those molecular sieves having an X-ray diffraction pattern including d-spacing maxima at 12.4±0.25, 6.9±0.15, 3.57±0.07 and 3.42±0.07 Angstrom. The X-ray diffraction data used to characterize the material are obtained by standard techniques using the K-alpha doublet of copper as incident radiation and a diffractometer equipped with a scintillation counter and associated computer as the collection system.

Examples of crystalline microporous materials of the MWW framework type include MCM-22 (described in U.S. Patent No. 4,954,325), PSH-3 (described in U.S. Patent No. 4,439,409), SSZ-25 (described in U.S. Patent No. 4,826,667), ERB-1 (described in European Patent No. 0293032), ITQ-1 (described in U.S. Patent No 6,077,498), ITQ-2 (described in International Patent Publication No. WO97/17290), MCM-36 (described in U.S. Patent No. 5,250,277), MCM-49 (described in U.S. Patent No. 5,236,575), MCM-56 (described in U.S. Patent No. 5,362,697), UZM-8 (described in U.S. Patent No. 6,756,030), UZM-8HS (described in U.S. Patent No. 7,713,513), UZM-37 (described in U.S. Patent No. 7,982,084; EMM-10 (described in U.S. Patent No. 7,842,277), EMM-12 (described in U.S. Patent No. 8,704,025), EMM-13 (described in U.S. Patent No. 8,704,023), MIT-1 (described by Luo et al in Chem. Sci., 2015, 6, 6320-6324), and mixtures thereof, with MCM-49 generally being preferred.

In some embodiments, the crystalline microporous material of the MWW framework type employed herein may be an aluminosilicate material having a silica to alumina molar ratio of at least 10, such as at least 10 to less than 50.

In some embodiments, the crystalline microporous material of the MWW framework type employed herein may be contaminated with other crystalline materials, such as ferrierite or quartz. These contaminants may be present in quantities < 10% by weight, normally < 5% by weight.

Other binder materials, including other inorganic oxides than alumina, such as silica, titania, zirconia and mixtures and compounds thereof, may be present in the solid acid catalyst used herein in amounts up to 90 wt%, for example up 80 wt%, such as up to 70 wt%, for example up to 60 wt%, such as up to 50 wt%. Where a non-alumina binder is present, the amount employed may be as little as 1 wt%, such as at least 5 wt%, for example at least 10 wt%. In one embodiment, a silica binder is employed such as disclosed in U.S. Patent No. 5,053,374. In other embodiments, a zirconia or titania binder is used.

In other embodiments, the crystalline microporous material is self-bound, that is substantially free of any inorganic oxide binder, although in some cases a temporary organic binder may be added to assist in forming the catalyst into the required shape. In such cases, the binder may be removed, such as by heating, before the catalyst is employed in the present alkylation process.

In other embodiments, the binder may be a crystalline oxide material such as the zeolite-bound-zeolites described in U.S. Patent Nos. 5,665,325 and 5,993,642. In the case of crystalline binders, the binder material may contain alumina.

Feedstocks useful in the present alkylation process include at least one isoparaffin and at least one olefin. The isoparaffin reactant used in the present alkylation process may have from about 4 to about 8 carbon atoms. Representative examples of such isoparaffins include isobutane, isopentane, 3-methylhexane, 2-methylhexane, 2,3-dimethylbutane, 2,4-dimethylhexane and mixtures thereof, especially isobutane.

The olefin component of the feedstock is selected from the group consisting of pentenes.

Isoparaffin to olefin ratios in the reactor feed typically range from about 1.5:1 to about 100:1, such as 10:1 to 75:1, measured on a volume to volume basis, so as to produce a high quality alkylate product at industrially useful yields. Higher isoparaffin:olefin ratios may also be used, but limited availability of produced isoparaffin within many refineries coupled with the relatively high cost of purchased isoparaffin favor isoparaffin:olefin ratios within the ranges listed above.

Before being sent to the alkylation reactor, the isoparaffin and/or olefin may be treated to remove catalyst poisons e.g., using guard beds with specific absorbents for reducing the level of S, N, and/or oxygenates to values which do not affect catalyst stability activity and selectivity.

The present alkylation process is suitably conducted at temperatures from about 275°F to about 700°F (135°C to 371°C), such as from about 300°F to about 600°F (149°C to 316°C). Operating temperature typically exceeds the critical temperature of the principal component in the feed. The term "principal component" as used herein is defined as the component of highest concentration in the feedstock. For example, isobutane is the principal component in a feedstock consisting of isobutane and 2-butene in isobutane:2-butene weight ratio of 50:1.

Operating pressure may similarly be controlled to maintain the principal component of the feed in the supercritical state, and is suitably from about 300 to about 1500 psig (2170 kPa-a to 10,445 kPa-a), such as from about 400 to about 1000 psig (2859 kPa-a to 6996 kPa-a). In some embodiments, the operating temperature and pressure remain above the critical value for the principal feed component during the entire process run, including the first contact between fresh catalyst and fresh feed.

Hydrocarbon flow through the alkylation zone containing the catalyst is typically controlled to provide an olefin liquid hourly space velocity (LHSV) sufficient to convert about 99 percent by weight of the fresh olefin to alkylate product. In some embodiments, olefin LHSV values fall within the range of about 0.01 to about 10 hr⁻¹.

The present isoparaffin-olefin alkylation process can be conducted in any known reactor, including reactors which allow for continuous or semi-continuous catalyst regeneration, such as fluidized and moving bed reactors, as well as swing bed reactor systems where multiple reactors are oscillated between on-stream mode and regeneration mode. Surprisingly, however, it is found that catalysts employing MWW framework type molecular sieves show unusual stability when used in isoparaffin-olefin alkylation. Thus, MWW-containing alkylation catalysts are particularly suitable for use in simple fixed bed reactors, without swing bed capability. In such cases, cycle lengths (on-stream times between successive catalyst regenerations) in excess of 150 days may be obtained.

The product composition of the isoparaffin-olefin alkylation reaction described herein is highly dependent on the reaction conditions and the composition of the olefin and isoparaffin feedstocks. In any event, the product is a complex mixture of hydrocarbons because alkylation of the feed isoparaffin by the feed olefin is accompanied by a variety of competing reactions including cracking, olefin oligomerization and further alkylation of the alkylate product by the feed olefin. For example, in the case of alkylation of isobutane with C₅ olefins, particularly n-pentene and 2-methyl-2-butene, the product may comprise about 30-40 wt% of C₅ hydrocarbons, 20-35 wt% of C₉ hydrocarbons, 10-15 wt% of octanes, and 15-25 wt% of C₁₀+ hydrocarbons. Moreover, using an MWW type molecular sieve as the catalyst, it is found that the process is selective to desirable high octane components so that, in the case of alkylation of isobutane with C₅ olefins, the C₈ and C₉ fractions typically comprise a higher molar ratio of trimethyl isomers to dimethyl isomers, which is beneficial for increasing octane. For the C₈ fraction, the molar ratio of trimethylpentane to dimethylhexane can be at least 3, e.g. at least 4 or 5, or between 3 and 6. For the C₉ fraction, the molar ratio of trimethylhexane to dimethylheptane can be at least 1, e.g. at least 1.5 or between 1 and 2.

The chemistry associated with these reactions can be described as follows using an example where the olefin feed is pentene and the isoparaffin feed is iso-butane. The below reaction description concentrates only on certain products, namely C₅, C₈, and C₉ paraffins. It would be understood by a person of skill in the art that many more reaction networks will be taking place in the reactor as described in the paragraph above. Under sufficient alkylation conditions in the presence of a catalyst, olefins from the olefin feed (here, pentene) adsorbs onto the catalyst surface, wherein it reacts with a proton present at an active catalyst site to create C₅H₁₁⁺ carbenium ions. The C₅H₁₁⁺ carbenium ions react with iso-butane to produce C₄H₉⁺ carbenium ions and pentane. Additional pentene combines with C₄H₉⁺ carbenium ions to form C₉H₁₉⁺ carbenium ions, which can then itself undergo hydride transfer with isobutane to form C₉H₂₀ (e.g. trimethylhexane and/or dimethylheptane). The C₉H₁₉⁺ carbenium ions can then crack to form additional C₅H₁₁⁺ carbenium ions and butene. C₅H₁₁⁺ carbenium ions undergo hydride transfer with isobutane to form C₅ paraffins and C₄H₉⁺ carbenium ions. Butenes combine with C₄H₉⁺ carbenium ions to form C₈H₁₇⁺ carbenium ions, which can then itself undergo hydride transfer with isobutane to form C₈H₁₈ (e.g. trimethylpentane and/or dimethylhexane). Chemical equations describing the above reactions are provided below.

C₅H₁₀ + H⁺ ↔ C₅H₁₁⁺

C₅Hn⁺ + C₄H₁₀ → C₅H₁₂ + C₄H₉⁺

C₅H₁₀ + C₄H₉⁺ → C₉H₁₉⁺

C₉H₁₉⁺ + C₄H₁₀ → C₉H₂₀ + C₄H₉⁺

C₉H₁₉⁺ → C₅H₁₁⁺ + C₄H₈

C₅H₁₁⁺ + C₄H₁₀ → C₅H₁₂ + C₄H₉⁺

C₄H₈ + C₄H₉⁺ → C₈H₁₇⁺

C₈H₁₇ ⁺ + C₄H₁₀ → C₈H₁₈ + C₄H₉⁺

The product of the isoparaffin-olefin alkylation reaction is conveniently fed to a separation system, such as a distillation train, to recover the C₈ and C₉ fractions for use as a gasoline octane enhancer. Depending on alkylate demand, part of all of the remaining C₁₀₊ fraction can be recovered for use as a distillate blending stock or can be recycled to the alkylation reactor to generate more alkylate. In particular, it is found that MWW type molecular sieves are effective to crack the C₁₀₊ fraction to produce light olefins and paraffins which can react to generate additional alkylate product and thereby increase overall alkylate yield.

The disclosure will now be more particularly described with reference to the following non-limiting Examples and the accompanying drawings.

### Example 1 Preparation of 80wt% MCM-49/20wt% Alumina Catalyst

80 parts MCM-49 zeolite crystals are combined with 20 parts pseudoboehmite alumina, on a calcined dry weight basis. The MCM-49 and pseudoboehmite alumina dry powder are placed in a muller or a mixer and mixed for about 10 to 30 minutes. Sufficient water and 0.05% polyvinyl alcohol are added to the MCM-49 and alumina during the mixing process to produce an extrudable paste. The extrudable paste is formed into a 1/20th inch quadralobe extrudate using an extruder. After extrusion, the 1/20th inch quadralobe extrudate is dried at a temperature ranging from 250°F to 325°F (121 to 163°C). After drying, the dried extrudate is heated to 1000°F (538°C) under flowing nitrogen. The extrudate is then cooled to ambient temperature and humidified with saturated air or steam.

After humidification, the extrudate is ion exchanged with 0.5 to 1 N ammonium nitrate solution. The ammonium nitrate solution ion exchange is repeated. The ammonium nitrate exchanged extrudate is then washed with deionized water to remove residual nitrate prior to calcination in air. After washing the wet extrudate, it is dried. The exchanged and dried extrudate is then calcined in a nitrogen/air mixture to a temperature 1000°F (538°C).

### Example 2 Testing in Isobutane/Pentene Alkylation

The catalyst of Example 1 was subjected to alkylation testing of a mixture of isobutane and pentene having the following composition by weight:

| | |
|---|---|
| Iso-butane | ∼97% |
| n-pentene | ∼1.95% |
| 2-methyl-2-butene | ∼1.05% |

The reactor used in these experiments comprised a stainless steel tube having an internal diameter of 3/8 in, a length of 20.5 in and a wall thickness of 0.035in. A piece of stainless steel tubing 8¾ in. long x 3/8 in. external diameter and a piece of ¼ inch tubing of similar length were positioned in the bottom of the reactor (one inside of the other) as a spacer to position and support the catalyst in the isothermal zone of the furnace. A ¼ inch plug of glass wool was placed at the top of the spacer to keep the catalyst in place. A 1/8 inch stainless steel thermo-well was placed in the catalyst bed, long enough to monitor temperature throughout the catalyst bed using a movable thermocouple. The catalyst is loaded with a spacer at the bottom to keep the catalyst bed in the center of the furnace's isothermal zone.

The catalyst was then loaded into the reactor from the top. The catalyst bed typically contained about 4 gm of catalyst sized to 14-25 mesh (700 to 1400 micron) and was 10 cm. in length. A ¼ inch plug of glass wool was placed at the top of the catalyst bed to separate quartz chips from the catalyst. The remaining void space at the top of the reactor was filled with quartz chips. The reactor was installed in the furnace with the catalyst bed in the middle of the furnace at the pre-marked isothermal zone. The reactor was then pressure and leak tested typically at 300 psig (2170 kPa-a).

500 cc ISCO syringe pumps were used to introduce the feed to the reactor. Two ISCO pumps were used for pumping the iso-butane (high flow rate 10-250 cc/hr) and one ISCO pump for pumping the pentenes (0.01-5 cc/hr). A Grove "Mity Mite" back pressure controller was used to control the reactor pressure typically at 750 psig (5272 kPa-a). On-line GC analyses were taken to verify feed and the product composition. The feed was then pumped through the catalyst bed initially at an LHSV of 0.12 hr⁻¹, then at an LHSV of 0.07 hr⁻¹, and then 0.03 hr⁻¹ with the catalyst bed held at a reaction temperature of 150°C. The products exiting the reactor flowed through heated lines routed to GC then to three cold (5-7°C) collection pots in series. The non-condensable gas products were routed through a gas pump for analyzing the gas effluent. Material balances were taken at 24 hr intervals. Samples were taken for analysis. The material balance and the gas samples were taken at the same time while an on-line GC analysis was conducted for doing material balance.

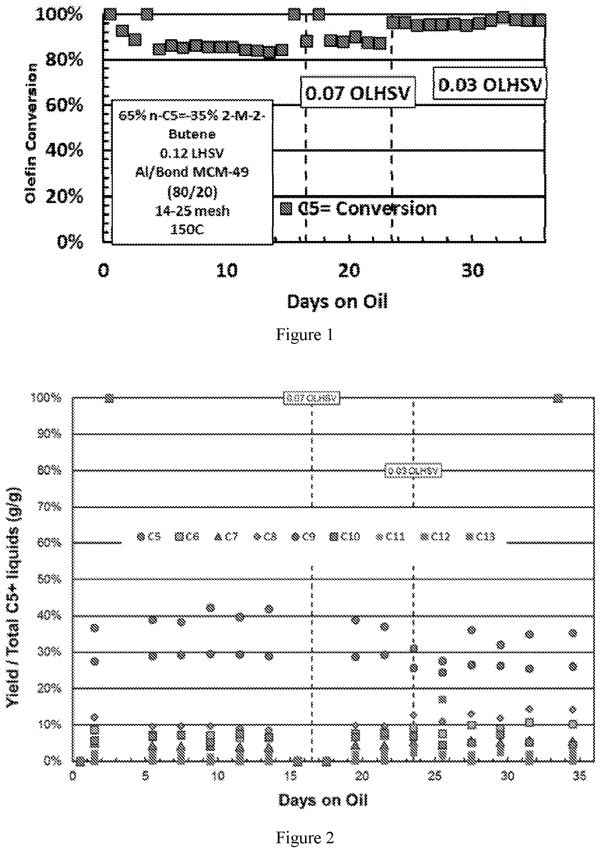

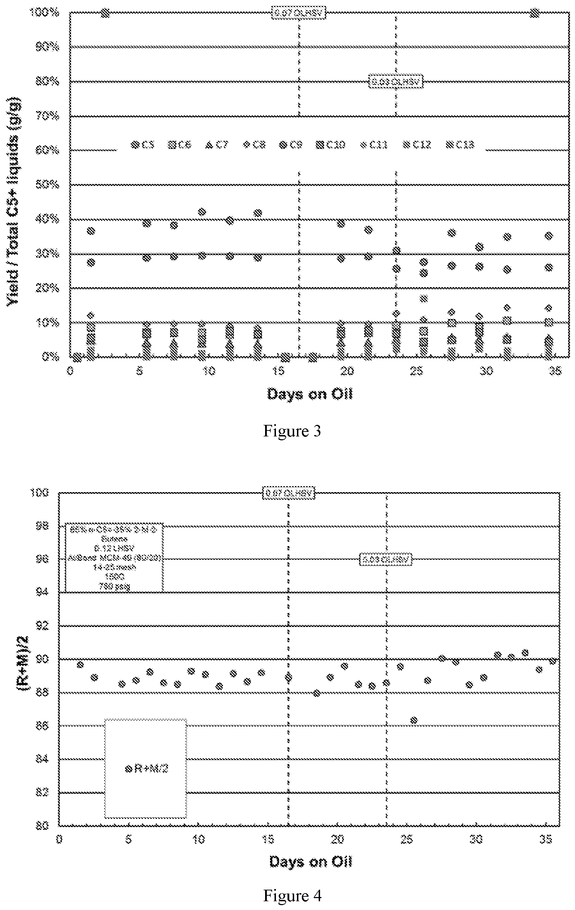

The results of the catalytic testing are shown in Figures 1 through 4. Figure 1 demonstrates that the MCM-49 catalyst has significantly high pentene conversion activity at varying LHSV at a constant reaction temperature of 150°C over a prolonged period of 35 days on stream. As shown, nearly 100% conversion can be obtained at a LHSV of 0.03 hr⁻¹. Figure 2 is a graph of C₅+ product yield broken out as a percentage of total C₅+ liquids. As can be seen, MCM-49 is a selective for catalyzing pentenes and iso-butylene with iso-butane (C₉ and C₈ alkylate accounts for about 30-40% of the yield). Figure 3 is a graph of the ratios of trimethyl versus dimethyl isomers in the C₇, C₈, and C₉ alkylate products. Trimethyl isomers have a higher octane number than dimethyl isomers, and therefore, are more desirable in this context. As shown, both C₈ and C₉ product produce significantly more trimethyl isomers than dimethyl isomers when using pentene as the olefin feed. Figure 4 shows that the octane number of the entire product stream is generally between 88 and 90 and is substantially constant over time.

### Example 3 Testing in Isobutane/n-pentene Alkylation

The catalyst of Example 1 was subjected to alkylation testing of a mixture of isobutane and pentene having the following composition by weight:

| | |
|---|---|
| Iso-butane | ∼97% |
| n-pentene | ∼3.0% |

The reactor used in these experiments comprised a stainless steel tube having an internal diameter of 3/8 in, a length of 20.5 in and a wall thickness of 0.035in. A piece of stainless steel tubing 8¾ in. long x 3/8 in. external diameter and a piece of ¼ inch tubing of similar length were positioned in the bottom of the reactor (one inside of the other) as a spacer to position and support the catalyst in the isothermal zone of the furnace. A ¼ inch plug of glass wool was placed at the top of the spacer to keep the catalyst in place. A 1/8 inch stainless steel thermo-well was placed in the catalyst bed, long enough to monitor temperature throughout the catalyst bed using a movable thermocouple. The catalyst is loaded with a spacer at the bottom to keep the catalyst bed in the center of the furnace's isothermal zone.

The catalyst was then loaded into the reactor from the top. The catalyst bed typically contained about 4 gm of catalyst sized to 14-25 mesh (700 to 1400 micron) and was 10 cm. in length. A ¼ inch plug of glass wool was placed at the top of the catalyst bed to separate quartz chips from the catalyst. The remaining void space at the top of the reactor was filled with quartz chips. The reactor was installed in the furnace with the catalyst bed in the middle of the furnace at the pre-marked isothermal zone. The reactor was then pressure and leak tested typically at 300 psig (2170 kPa-a).

500 cc ISCO syringe pumps were used to introduce the feed to the reactor. Two ISCO pumps were used for pumping the iso-butane (high flow rate 10-250 cc/hr) and one ISCO pump for pumping the pentenes (0.01-5 cc/hr). A Grove "Mity Mite" back pressure controller was used to control the reactor pressure typically at 750 psig (5272 kPa-a). On-line GC analyses were taken to verify feed and the product composition. The feed was then pumped through the catalyst bed initially at an LHSV of 0.06 hr⁻¹ with the catalyst bed held at a reaction temperature of 150°C. The products exiting the reactor flowed through heated lines routed to GC then to three cold (5-7°C) collection pots in series. The non-condensable gas products were routed through a gas pump for analyzing the gas effluent. Material balances were taken at 24 hr intervals. Samples were taken for analysis. The material balance and the gas samples were taken at the same time while an on-line GC analysis was conducted for doing material balance.

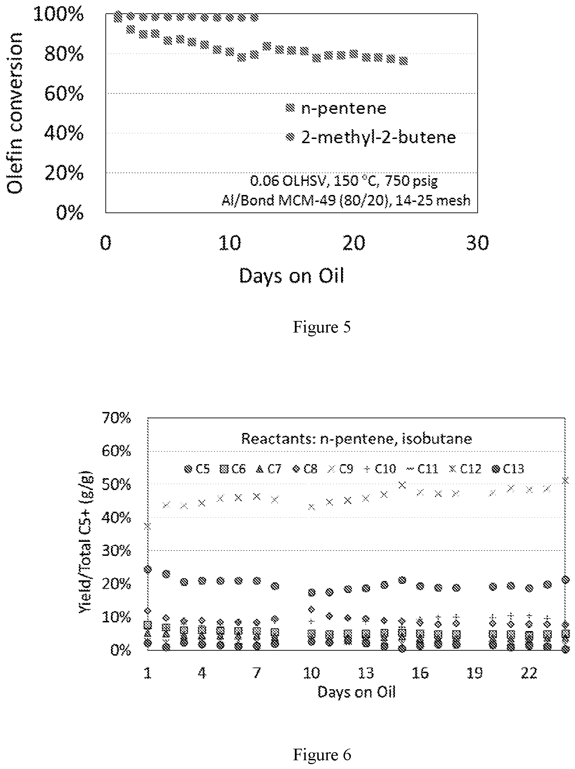

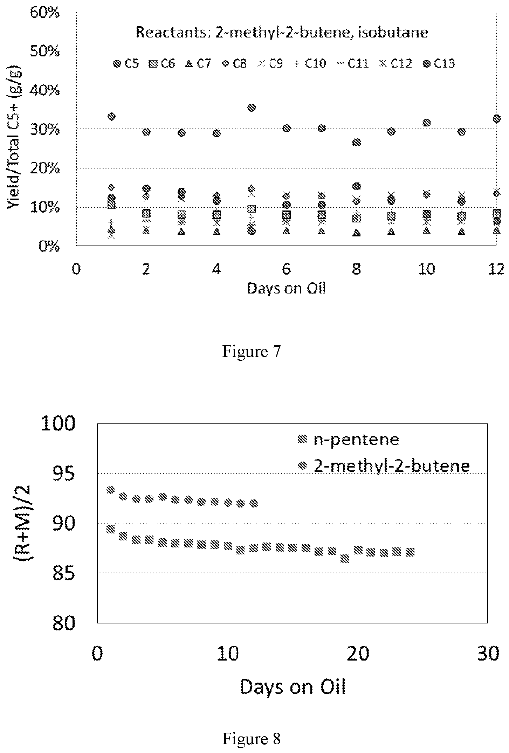

The results of the catalytic testing are shown in Figures 5, 6, and 8 through 4. Figure 5 demonstrates that the MCM-49 catalyst has significantly high n-pentene conversion activity at 0.06 LHSV at a reaction temperature of 150°C over a prolonged period of about 25 days on stream. As shown, nearly 100% conversion can be obtained at the beginning of the experiment, but that gradually tapers off to about 80% and remains nearly constant thereafter at about day 10. Figure 6 is a graph of C₅+ product yield broken out as a percentage of total C₅+ liquids. As can be seen, MCM-49 is a selective catalyst catalyzing the alkylation of n-pentene and iso-butylene with iso-butane (C₉ and C₈ alkylate accounts for about 50-60% of the yield, with C₉ dominating the yield at between 40-50%). Figure 8 shows that the octane number of the entire product stream is generally between 87 and 90 and is substantially constant over time.

### Example 4 Testing in Isobutane/2-methyl-2-butene Alkylation

The catalyst of Example 1 was subjected to alkylation testing of a mixture of isobutane and pentene having the following composition by weight:

| | |
|---|---|
| Iso-butane | ∼97% |
| 2-methyl-2-butene | ∼3.0% |

The reactor used in these experiments comprised a stainless steel tube having an internal diameter of 3/8 in, a length of 20.5 in and a wall thickness of 0.035in. A piece of stainless steel tubing 8¾ in. long x 3/8 in. external diameter and a piece of ¼ inch tubing of similar length were positioned in the bottom of the reactor (one inside of the other) as a spacer to position and support the catalyst in the isothermal zone of the furnace. A ¼ inch plug of glass wool was placed at the top of the spacer to keep the catalyst in place. A 1/8 inch stainless steel thermo-well was placed in the catalyst bed, long enough to monitor temperature throughout the catalyst bed using a movable thermocouple. The catalyst is loaded with a spacer at the bottom to keep the catalyst bed in the center of the furnace's isothermal zone.

The catalyst was then loaded into the reactor from the top. The catalyst bed typically contained about 4 gm of catalyst sized to 14-25 mesh (700 to 1400 micron) and was 10 cm. in length. A ¼ inch plug of glass wool was placed at the top of the catalyst bed to separate quartz chips from the catalyst. The remaining void space at the top of the reactor was filled with quartz chips. The reactor was installed in the furnace with the catalyst bed in the middle of the furnace at the pre-marked isothermal zone. The reactor was then pressure and leak tested typically at 300 psig (2170 kPa-a).

500 cc ISCO syringe pumps were used to introduce the feed to the reactor. Two ISCO pumps were used for pumping the iso-butane (high flow rate 10-250 cc/hr) and one ISCO pump for pumping the pentenes (0.01-5 cc/hr). A Grove "Mity Mite" back pressure controller was used to control the reactor pressure typically at 750 psig (5272 kPa-a). On-line GC analyses were taken to verify feed and the product composition. The feed was then pumped through the catalyst bed initially at an LHSV of 0.06 hr⁻¹ with the catalyst bed held at a reaction temperature of 150°C. The products exiting the reactor flowed through heated lines routed to GC then to three cold (5-7°C) collection pots in series. The non-condensable gas products were routed through a gas pump for analyzing the gas effluent. Material balances were taken at 24 hr intervals. Samples were taken for analysis. The material balance and the gas samples were taken at the same time while an on-line GC analysis was conducted for doing material balance.

The results of the catalytic testing are shown in Figures 5, 7, and 8 through 4. Figure 5 demonstrates that the MCM-49 catalyst has significantly high 2-methyl-2-butene conversion activity at 0.06 LHSV at a reaction temperature of 150°C over a prolonged period of about 12 days on stream. As shown, nearly 100% conversion and that conversion rate remains fairly constant throughout. Figure 7 is a graph of C₅+ product yield broken out as a percentage of total C₅+ liquids. As can be seen, MCM-49 is a selective catalyst for catalyzing the alkylation of iso-pentene and iso-butylene with iso-butane (C₉ and C₈ alkylate accounts for about 20-30% of the yield). Perhaps more significantly the ratio of trimethyl isomers to dimethyl isomers for C₈ compounds was 21.5:1 (trimethylpentane:dimethylhexane). Figure 8 shows that the octane number of the entire product stream is generally between 91 and 94 and is substantially constant over time.

## Claims

1. A process for the catalytic alkylation of an olefin with an isoparaffin, the process comprising: contacting an olefin-containing feed with an isoparaffin-containing feed under alkylation conditions in the presence of a solid acid catalyst comprising a crystalline microporous material of the MWW framework type, wherein the olefin-containing feed consists essentially of pentenes.

2. The process of claim 1, wherein the olefin-containing feed consists essentially of n-pentene, iso-pentene, or a combination thereof.

3. The process of claim 1, wherein the solid acid catalyst comprises an inorganic oxide binder.

4. The process of claim 1, wherein the solid acid catalyst comprises at least one of alumina, silica, titania, and zirconia binder.

5. The process of claim 1, wherein the crystalline microporous material of the MWW framework type is selected from the group consisting of MCM-22, PSH-3, SSZ-25, ERB-1, ITQ-1, ITQ-2, MCM-36, MCM-49, MCM-56, EMM-10, EMM-12, EMM-13, UZM-8, UZM-8HS, UZM-37, MIT-1, and mixtures thereof.

6. The process of claim 1, wherein the crystalline microporous material of the MWW framework type comprises MCM-49.

7. The process of claim 1, wherein the isoparaffin-containing feed comprises at least one C₄ to C₈ isoparaffin.

8. The process of claim 1, wherein the isoparaffin-containing feed comprises isobutane.

9. A hydrocarbon product produced by the process of any one of claims 1 to 8, said hydrocarbon product comprising a C₈ fraction comprising trimethylpentane isomers and dimethylhexane isomers and a C₉ fraction comprising trimethylhexane isomers and dimethylheptane isomers, wherein the ratio of trimethylpentane isomers to dimethylhexane isomers is from 3:1 to 22:1 in the C₈ fraction, wherein the ratio of trimethylhexane isomers to dimethylheptane isomers is from 0.5:1 to 2:1 in the C₉ fraction.

10. The hydrocarbon product of claim 9, further comprising an isoparaffin C₅ fraction that makes up between 18-45 wt% of the total C₅₊ liquids in the hydrocarbon product.

11. The hydrocarbon product of claim 9, further comprising a C₉ fraction that makes up between 10-50 wt% of the total C₅₊ liquids in the hydrocarbon product.

## Patentansprüche

1. Verfahren zur katalytischen Alkylierung von einem Olefin mit einem Isoparaffin, wobei bei dem Verfahren ein olefinhaltiges Einsatzmaterial unter Alkylierungsbedingungen in Gegenwart eines Festkörpersäurekatalysators, der ein kristallines mikroporöses Material der MWW-Gerüststruktur enthält, mit einem isoparaffinhaltigen Einsatzmaterial in Kontakt gebracht wird, wobei das olefinhaltige Einsatzmaterial im Wesentlichen aus Pentenen besteht.

2. Verfahren nach Anspruch 1, bei dem das olefinhaltige Einsatzmaterial im Wesentlichen aus n-Penten, Isopenten oder einer Kombination davon besteht.

3. Verfahren nach Anspruch 1, bei dem der Festkörpersäurekatalysator ein anorganisches Oxidbindemittel enthält.

4. Verfahren nach Anspruch 1, bei dem der Festkörpersäurekatalysator mindestens eines von Aluminiumoxid-, Siliziumdioxid-, Titandioxid- und Zirkonoxid-Bindemittel enthält.

5. Verfahren nach Anspruch 1, bei dem das kristalline mikroporöse Material der MWW-Gerüststruktur ausgewählt ist aus der Gruppe bestehend aus MCM-22, PSH-3, SSZ-25, ERB-1, ITQ-1, ITQ-2, MCM-36, MCM-49, MCM-56, EMM-10, EMM-12, EMM-13, UZM-8, UZM-8HS, UZM-37, MIT-1 und Mischungen davon.

6. Verfahren nach Anspruch 1, bei dem das kristalline mikroporöse Material der MWW-Gerüststruktur MCM-49 enthält.

7. Verfahren nach Anspruch 1, bei dem das isoparaffinhaltige Einsatzmaterial mindestens ein C₄- bis Cs-Isoparaffin enthält.

8. Verfahren nach Anspruch 1, bei dem das isoparaffinhaltige Einsatzmaterial Isobutan enthält.

9. Kohlenwasserstoffprodukt, das durch ein Verfahren nach einem der Ansprüche 1 bis 8 hergestellt ist, wobei das Kohlenwasserstoffprodukt eine C₈-Fraktion, die Trimethylpentanisomere und Dimethylhexanisomere enthält, und eine C₉-Fraktion, die Trimethylhexanisomere und Dimethylheptanisomere enthält, umfasst, wobei das Verhältnis von Trimethylpentanisomeren zu Dimethylhexanisomeren in der C₈-Fraktion von 3:1 bis 22:1 beträgt und wobei das Verhältnis von Trimethylhexanisomeren zu Dimethylheptanisomeren in der C₉-Fraktion von 0,5:1 bis 2:1 beträgt.

10. Kohlenwasserstoffprodukt nach Anspruch 9, das ferner eine C₅-Isoparaffinfraktion enthält, die zwischen 18 und 45 Gew.-% der gesamten C₅₊-Flüssigkeiten im Kohlenwasserstoffprodukt ausmacht.

11. Kohlenwasserstoffprodukt nach Anspruch 9, das ferner eine Cg-Fraktion enthält, die zwischen 10 und 50 Gew.-% der gesamten C₅₊-Flüssigkeiten im Kohlenwasserstoffprodukt ausmacht.

## Revendications

1. Procédé d'alkylation catalytique d'une oléfine avec une isoparaffine, le procédé comprenant : la mise en contact d'une charge contenant une oléfine avec une charge contenant de l'isoparaffine dans des conditions d'alkylation en présence d'un catalyseur acide solide comprenant un matériau microporeux cristallin du type à structure MWW, dans lequel la charge contenant une oléfine est essentiellement constituée de pentènes.

2. Procédé selon la revendication 1, dans lequel la charge contenant une oléfine est essentiellement constituée du n-pentène, de l'iso-pentène, ou une combinaison de ceux-ci.

3. Procédé selon la revendication 1, dans lequel le catalyseur acide solide comprend un liant à base d'oxyde inorganique.

4. Procédé selon la revendication 1, dans lequel le catalyseur acide solide comprend au moins l'un parmi un liant d'alumine, de silice, d'oxyde de titane et de zircone.

5. Procédé selon la revendication 1, dans lequel le matériau microporeux cristallin du type de structure MWW est choisi dans le groupe constitué de MCM-22, PSH-3, SSZ-25, ERB-1, ITQ-1, ITQ-2, MCM-36, MCM-49, MCM-56, EMM-10, EMM-12, EMM-13, UZM-8, UZM-8HS, UZM-37, MIT-1, et des mélanges de ceux-ci.

6. Procédé selon la revendication 1, dans lequel le matériau microporeux cristallin du type de structure MWW comprend MCM-49.

7. Procédé selon la revendication 1, dans lequel la charge contenant de l'isoparaffine comprend au moins une isoparaffine en C₄ à C₈.

8. Procédé selon la revendication 1, dans lequel la charge contenant de l'isoparaffine comprend de l'isobutane.

9. Produit hydrocarbure produit par le procédé selon l'une quelconque des revendications 1 à 8, ledit produit hydrocarbure comprenant une fraction en C₈ comprenant des isomères de triméthylpentane et des isomères de diméthylhexane et une fraction en C₉ comprenant des isomères de triméthylhexane et des isomères de diméthylheptane, dans lequel le rapport des isomères de triméthylpentane aux isomères de diméthylhexane est de 3:1 à 22:1 dans la fraction en C₈, dans lequel le rapport des isomères de triméthylhexane aux isomères de diméthylheptane est de 0,5:1 à 2:1 dans la fraction en C₉.

10. Produit hydrocarbure selon la revendication 9, comprenant en outre une fraction en C₅ d'isoparaffine qui constitue entre 18 et 45 % en poids des liquides en C₅₊ totaux dans le produit hydrocarbure.

11. Produit hydrocarbure selon la revendication 9, comprenant en outre une fraction C₉ qui constitue entre 10 et 50 % en poids des liquides en C₅₊ totaux dans le produit hydrocarbure.
